# EUROPEAN PATENT SPECIFICATION

(11) **EP 3 328 286 B1**
(45) Date of publication and mention of the grant of the patent: **11.09.2019**
(21) Application number: 16750103.0
(22) Date of filing: 29.07.2016
(51) Int. Cl.: A61B 10/00

(54) **DISPOSABLE CONTAINER OF 30 OR 90 ML FOR THE STORAGE AND TRANSPORTATION OF HUMAN TISSUE SAMPLES OR ORGANS TO BE SUBJECTED TO HISTOLOGICAL EXAMINATION**
EINWEGKONTAINER DER GRÖSSE 30 ODER 90ML GEEIGNET ZUR AUFBEWAHRUNG ODER ZUM TRANSPORT VON MENSCHLICHEM GEWEBE ODER ORGANEN, DIE EINER HISTOLOGISCHEN UNTERSUCHUNG UNTERZOGEN WERDEN SOLLEN
RECIPIENT DE 30 OU 90ML POUR STOCKAGE OU TRANSPORT DE TISSUS OU ORGANES HUMAINS EN VUE D'UN EXAMINATION HISTOLOGIQUE

(30) Priority: 30.07.2015 IT UB20152656
(43) Date of publication of application: 06.06.2018
(73) Proprietor: Tecnobilife S.r.l., 57121 Livorno (IT)
(72) Inventor: CINTI, Mario, 63100 Ascoli Piceno (AP) (IT); BICOCCHI, Enrico, 57128 Livorno (LI) (IT)
(74) Representative: Primiceri, Maria Vittoria
(86) International application number: PCT/EP2016/025082
(87) International publication number: WO 2017/016676

(56) References cited:
- WO-A1-2013/192606
- US-A1- 2011 060 137
- US-A1- 2015 037 830

## Description

### Field of the invention

This invention relates to the field of histology and, more specifically, that of preservation by means of formalin of human tissues or organs to be subjected to histological examination and consists of a disposable container of 30 or 90 ml for the preservation and transport of human tissue samples or organs or parts thereof, comprising: a receptacle with a buffer solution, a piercing cap, a capsule containing formalin sealed by a protective film and a safety ring nut provided with a stopper.

### Background of the invention

In hospitals and clinics, containers and Petri dishes are known and used for the collection, preservation and transport of human tissue samples or organs, or parts thereof, to be subjected to histological examination.

According to the known art, a tissue sample or organ, or parts thereof, taken from a patient is placed in a receptacle that is subsequently filled with a preservative, preferably formalin, closed and sent to a laboratory for analysis. The current 30- and 90-ml containers, packed with 4% buffered formalin and closed with the screw cap, have the drawback that the operator must open the container under a chemical or extraction hood to protect himself from the formalin fumes, which are toxic. For this reason, in the absence of said hood, when handling human tissue samples or organs, or parts thereof, operators must compulsorily use personal protective equipment such as face masks for organic vapours, a non-woven fabric head-covering, protective gloves for chemical reagents, protective goggles or a face shield, shoes with waterproof ankle pads and a disposable apron.

It follows that all personnel involved in taking tissue samples or organs, or parts thereof, come in contact with formalin, with the risk of intoxication.

To overcome this drawback, some hospital facilities use a formalin dispensing station or a dispenser mountable on the container that, once filled with formalin, it is closed by screwing a lid.

The drawback of this procedure is that, during the opening and closing of the container, the formalin may accidentally spill, exposing operators to its toxic vapours.

Another drawback concerns the known method for packaging biopsies because the needle of the device used for taking tissue, organs, or parts thereof, to be examined cannot be introduced inside the container itself since formalin is present. It follows that, the detachment of the biopsy sample, immersed in formalin, from the cavity of the needle of the device must take place outside of the container with obvious risks of contamination of those assigned to perform these operations.

A further drawback of the known tissue or human organ containers as described above is the difficulty of introducing other devices inside them, such as the disposable plastic box that the operator uses when the biopsy taken must be oriented to facilitate subsequent diagnosis in the laboratory. Said box, for reasons of lack of space in the container, must be positioned vertically and not horizontally with the consequence that the human tissue or organs, or parts thereof, are not fully covered by the formalin.

In the state of the art, there is known patent WO2012171529 which discloses a container comprising a receptacle for the preservation of a human tissue sample and a lid suitable to engage with the receptacle and comprising: a receptacle with, as its upper element, a gasket for sealing said container and a piercing element for breaking said gasket.

The drawbacks of the object of the application cited above are:
- the lack of a safety for crushing the cap;
- the circumstance that, during transport, the container is placed in horizontal position or oblique position and the formalin or preservative fluid, entering the cap or inside its holes, does not return into the container and does not entirely cover the sample of human tissue to be preserved when the container is repositioned vertically.

In the state of the art the following documents are known:
- US 2011/060137 A1 which discloses a container and relative method for processing a stool sample;
- US 2015/037830 A1 which discloses a container and relative method for storing a tissue sample;
- WO 2013/192606 A1 which discloses a device for the transport of a biopsy tissue sample and method of using thereof.

From the documents cited above, in the state of the art the problem of accidental breakage of the protective film of the formalin contained in the capsule.

### Disclosure of the invention

The purpose of this invention is the design of a disposable container for the preservation and transport of samples of human tissue or organs, or parts thereof, to be subjected to histological examination that eliminates and, in any case, reduces the risk of exposure of the operators to formalin during their transport from one department of a hospital to the other and during the operations of their insertion and withdrawal from the container.

Another purpose of this invention is the design of a disposable container for the preservation and transport of a sample of human tissue or organs to be subjected to histological examination that allows introducing inside it the device for detaching the biopsy material.

Another purpose of this invention is the design of a disposable container for the preservation and transport of a sample of human tissue or organs to be subjected to histological examination that allows positioning the disposable plastic box horizontally so as to have the entire human tissue or organ contained in it constantly covered by the formalin, ensuring its complete preservation.

These and other purposes are achieved with this invention which is defined in the appended claims.

Further characteristics and advantages of the invention will become apparent from the description of a preferred, but not exclusive, embodiment of the container covered by this patent application, illustrated by way of non-limiting indication in the accompanying drawings in which:

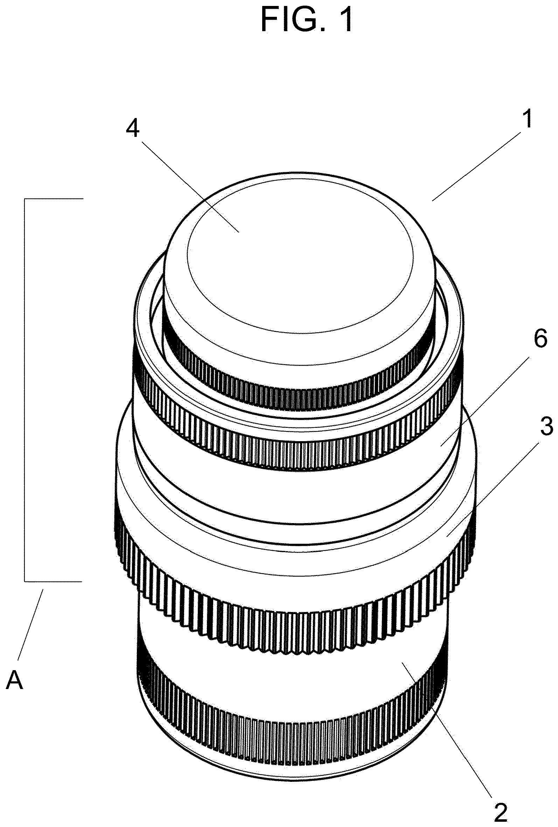

- Fig. 1 shows, in a three-dimensional view, a container (1) of 90 ml consisting of:
   - a receptacle (2);
   - a piercing cap (3);
   - a capsule (4);
   - a safety ring nut (6);
   - a stopper (7) (not visible);
   and where A is considered the set of the piercing cap (3), the capsule (4) and the safety ring nut (6);

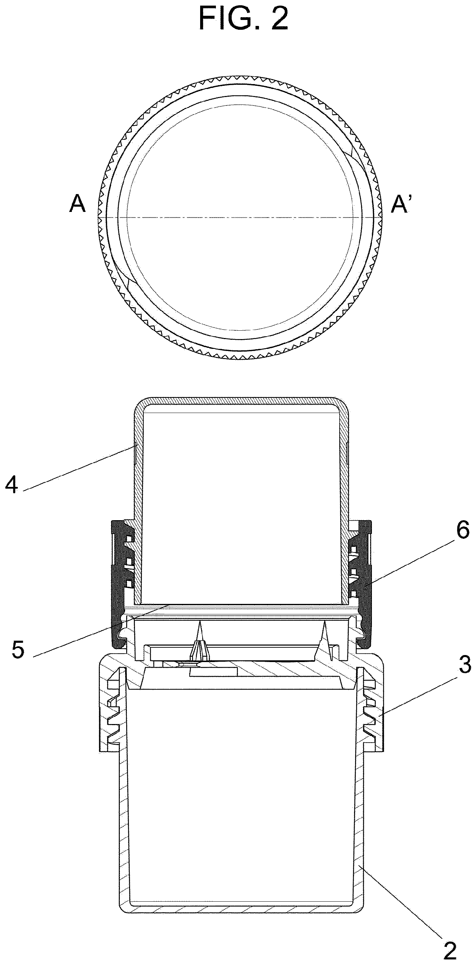

- Fig. 2 shows, both from above and in a sectioned view, along the section line A-A', the container (1) of 90 ml in the initial position, i.e., the moment in which the piercing cap (3) has not yet pierced the protective film (5);

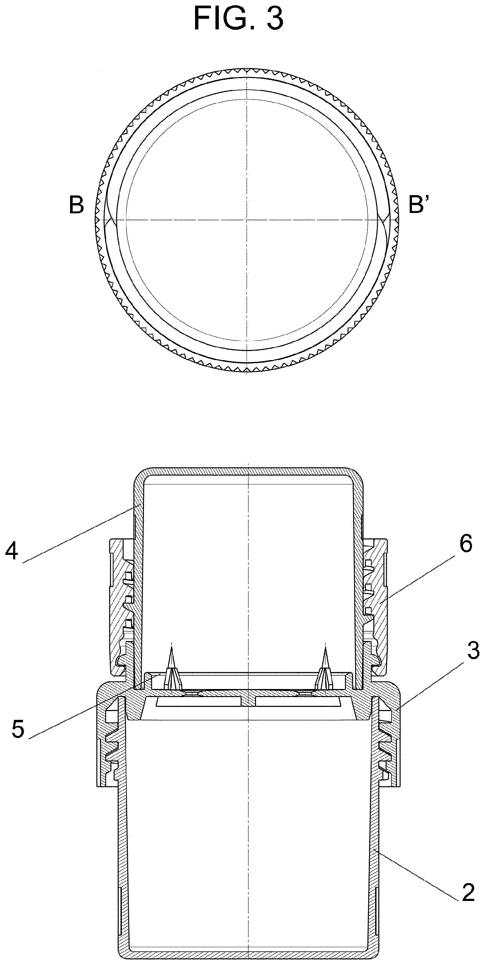

- Fig. 3 shows, both from above and in a sectioned view, along the section line B-B', the container (1) of 90 ml in the final position, i.e., the moment in which the piercing cap (3) has pierced the protective film (5);

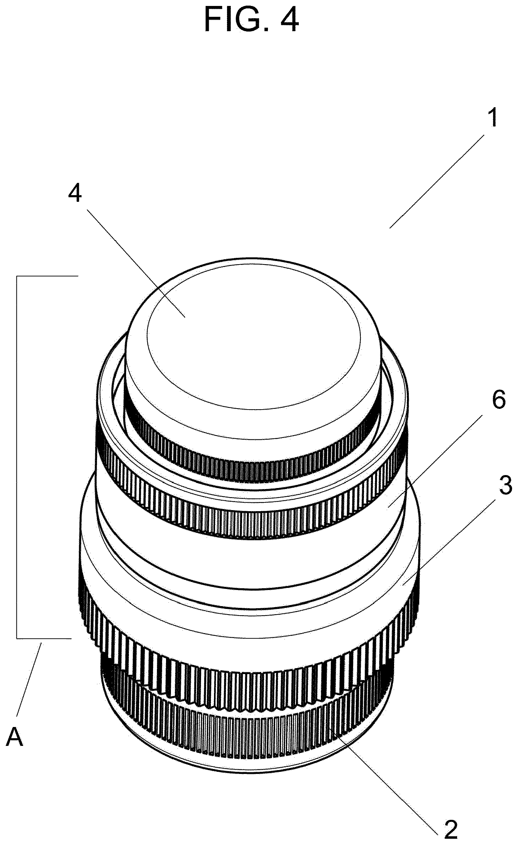

- Fig. 4 shows, in a three-dimensional view, a container (1) of 30 ml consisting of:
   - a receptacle (2);
   - a piercing cap (3);
   - a capsule (4);
   - a safety ring nut (6);
   - a stopper (7) (not visible);
   and where A is considered the set of the piercing cap (3), the capsule (4) and the safety ring nut (6);

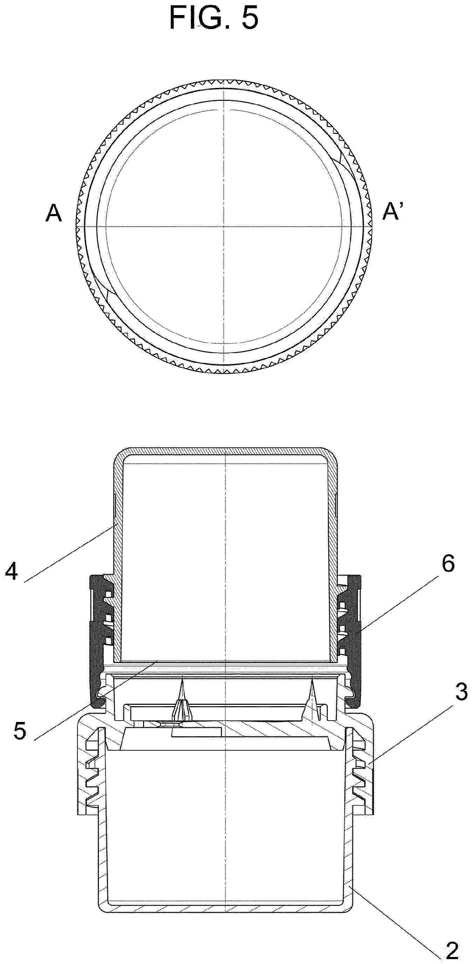

- Fig. 5 shows, both from above and in a sectioned view, along the section line A-A', the container (1) of 30 ml in the initial position, i.e., the moment in which the piercing cap (3) has not yet pierced the protective film (5);

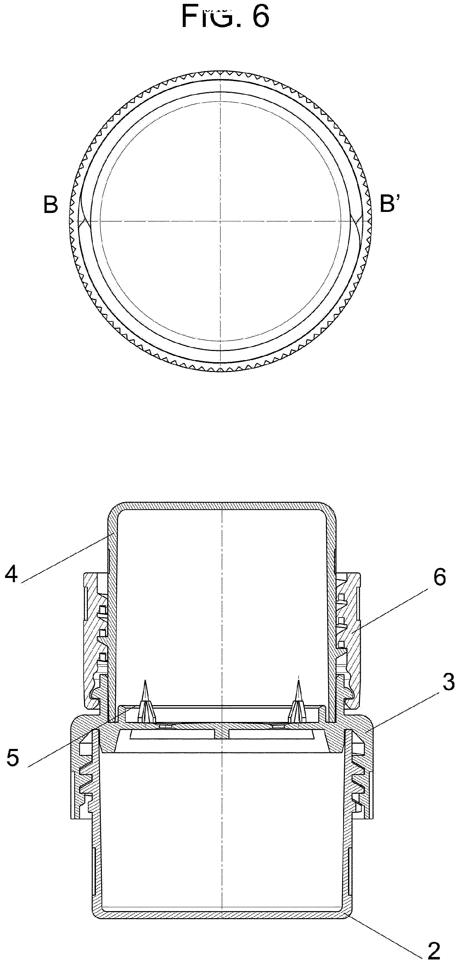

- Fig. 6 shows, both from above and in a sectioned view, along the section line B-B', the container (1) of 30 ml in the final position, i.e., the moment in which the piercing cap (3) has pierced the protective film (5);

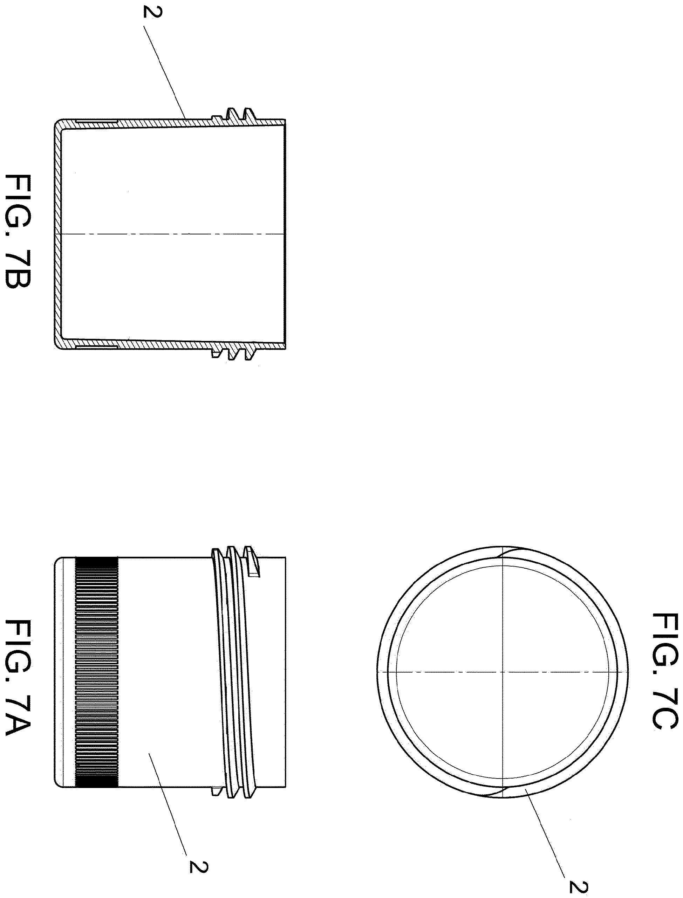

- Fig. 7A shows, in a side view, the receptacle (2) of 90 ml;
- Fig. 7B shows, in a sectioned view, the receptacle (2) of the container (1) of 90 ml;
- Fig. 7C shows, from above, the receptacle (2) of the container (1) of 90 ml;

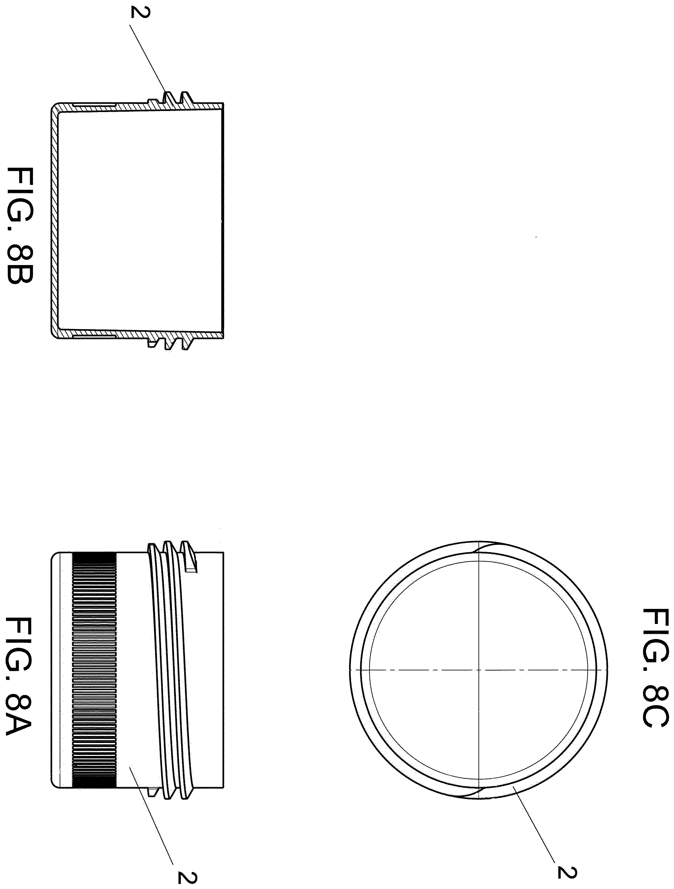

- Fig. 8A shows, in a side view, the receptacle (2) of the container (1) of 30 ml;
- Fig. 8B shows, in a sectioned view, the receptacle (2) of the container (1) of 30 ml;
- Fig. 8C shows, from above, the receptacle (2) of the container (1) of 30 ml;

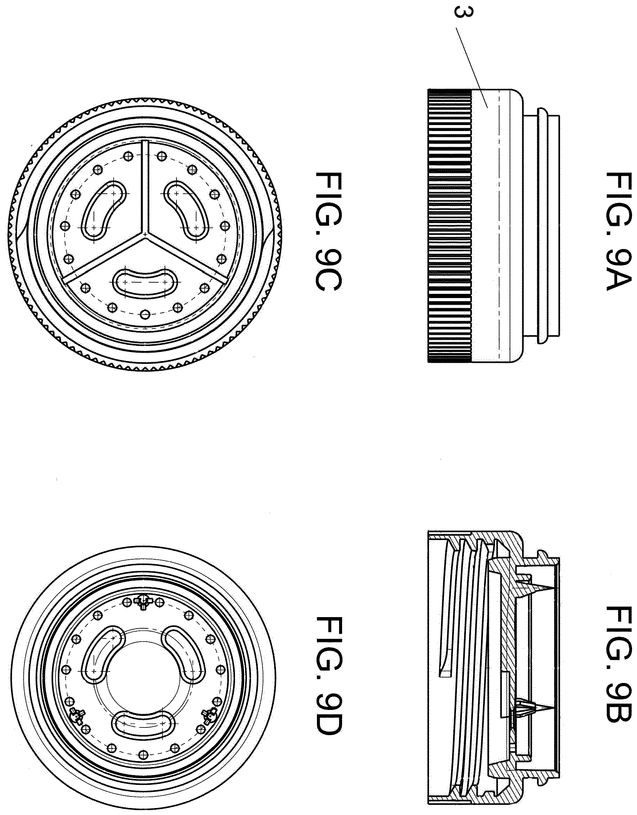

- Fig. 9A shows, in a side view, the piercing cap (3) of a container (1);
- Fig. 9B shows, in a sectioned view, the piercing cap (3) of a container (1);
- Fig. 9C shows, in a view from the bottom up, the piercing cap (3) of a container (1);
- Fig. 9D shows, in a view from the top down, the piercing cap (3) of a container (1);

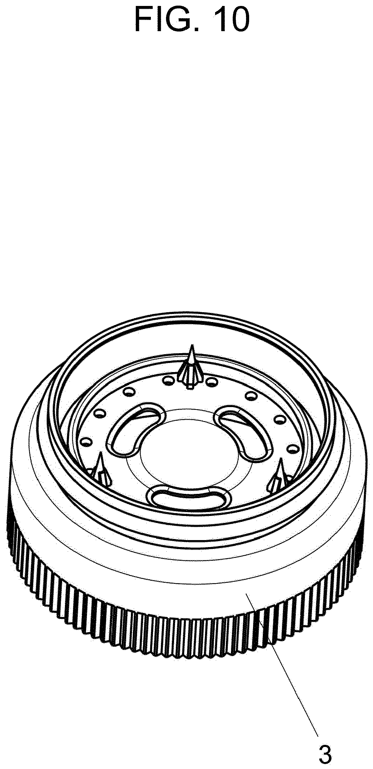

- Fig. 10 shows, in a three-dimensional view, the piercing cap (3);

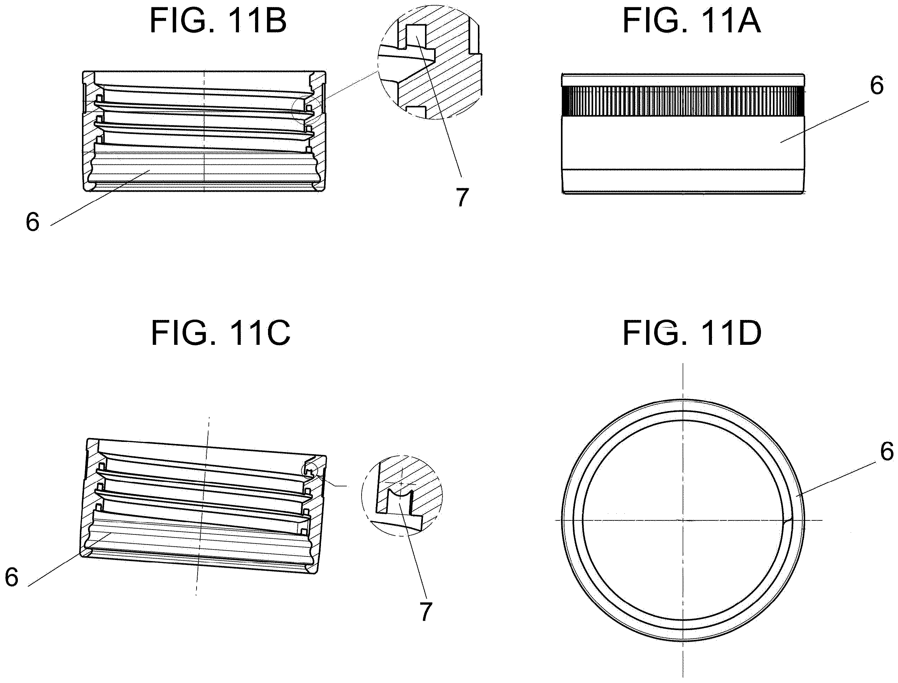

- Fig. 11A shows, in a side view, the safety ring nut (6) of a container (1);
- Fig. 11B shows, in a sectioned view, the safety ring nut (6) of a container (1) and, in particular, the stopper (7);
- Fig. 11C shows, in a further sectioned view, the safety ring nut (6) of a container (1) and, in particular, the stopper (7);
- Fig. 11D shows, in a view from above, the safety ring nut (6) of a container (1);

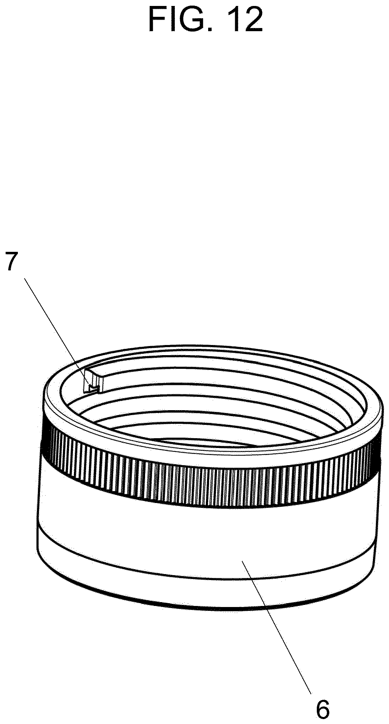

- Fig. 12 shows, in a three-dimensional view, the safety ring nut (6) of a container (1) in which the stopper (7) is visible;

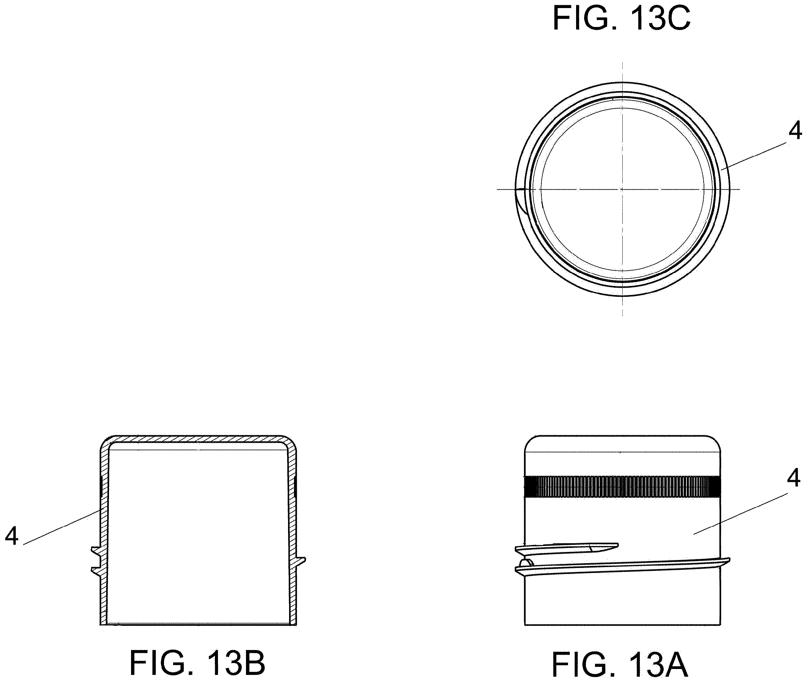

- Fig. 13A shows, in a side view, the capsule (4) of a container (1);
- Fig. 13B shows, in a sectioned view, the capsule (4) of a container (1);
- Fig. 13C shows, in a view from above, the capsule (4) of a container (1).

### Detailed description of the Invention

According to a preferred, but non-limiting, embodiment, this invention relates to a disposable container (1) of 30 or 90 ml for the preservation and transport of a sample of human tissue or organs, or parts thereof, to be subjected to histological examination comprising: a receptacle (2) with buffer solution, a piercing cap (3), a capsule (4) containing formalin sealed by a protective film (5) and, finally, a safety ring nut (6) provided with a stopper (7).

The container (1) covered by this patent application has the formalin diluted to 40% and 1/10 of the buffer solution contained in the capsule (4), which is sealed by means of a protective film (5).

To pass from the 40% dilution to 4%, the formalin must be combined with other buffer solution contained in the receptacle (2) of the container (1). Buffer solution means a solution that opposes the change of pH due to moderate additions of acids or bases.

The buffer solution, present in the receptacle (2), facilitates the detachment of the patient sample from the device, because the receptacle (2) is not empty as is instead in the case of the containers of the known art.

In the specific case, in the containers (1) of 90 ml, the capsule (4) contains 9 ml of formalin and 1/10 of the total buffer solution, which is 81 ml. Therefore, in the capsule (4) there is 17.1 ml of solution.

To reach the total of 90 ml, the buffer solution contained inside the receptacle (2) is of 72.9 ml; in this way the formalin will pass from a concentration of 40% to a concentration of 4%.

While, in the case of the containers (1) of 30 ml, the capsule (4) contains 3 ml of formalin and 1/10 of the total buffer solution, which is 27 ml.

Therefore, in the capsule (4) there is 5.7 ml of solution.

To reach the total of 30 ml, the buffer solution contained inside the receptacle (2) is of 24.3 ml; in this way the formalin, in a manner entirely similar to that described above, will pass from a concentration of 40% to a concentration of 4%.

In this patent application, as a preferred embodiment, we have chosen that of the containers (1) of 30 to 90 ml, however, said container (1) could be realised with an intermediate capacity between 30 and 90 ml, since the quantity of formalin and buffer solution would vary proportionally according to the principle described above.

The necessarily having to dilute the formalin from 40% to 4% is due to the fact that if, in the receptacle (2) the formalin was 40% and oncological tissue were introduced, the latter would suffer damage at the cellular level.

The receptacles (2) of 30 or 90 ml can be made of polypropylene but also polyethylene. Said receptacles (2) have the same diameter, which in the preferred, but not limiting, embodiment is 52 mm, and different variable heights: between 55 mm for a container (1) of 90 ml; 40 mm for a container (1) of 30 ml, for their different containing capacity.

The container (1) comprises a ring nut (6) positioned between the base of the piercing cap (3) and the capsule (4), having the function of preventing the accidental breakage of the protective film (5) adhering to the capsule (4) with consequent descent of the formalin liquid into the receptacle (2).

In this embodiment, the piercing cap (3) is provided with threading that will pierce the protective film (5) at the moment when the capsule (4) is screwed down in correspondence of said piercing cap (3).

The piercing of the protective film (5) occurs in the moment in which the capsule (4), which is screwed onto the threading of the ring nut (6), will force, to breaking, the stopper (7) positioned on the threading of said ring nut (6).

The size of the receptacles (2) are such as to allow the introduction of other devices such as, for example, disposable plastic boxes, which can be positioned horizontally and thus be completely covered by the fixative "formalin".

The cap (A), i.e., the safety ring nut (6), together with the piercing cap (3) and the capsule (4), allows safely opening the receptacle (2) for the insertion of the human tissue sample or organ to be analysed, avoiding, due to the interposition of the ring nut (6), the accidental breakage of the protective film (5) adhering to the capsule (4) and preventing any spilling of the formalin.

The caps (A) of the containers (1) of both 30 and 90 ml will be of different colours to allow identification of the clinic of origin.

The preservation of an oncological tissue sample as described in this patent application, occurs with:
- the opening of the disposable container (1), which is achieved by unscrewing the receptacle (2) from the cap (A);
- the introduction inside the receptacle (2), containing the buffer solution, of the needle of the device with the human tissue sample or organ taken from the patient;
- the detachment of the human tissue sample or organ facilitated by the presence of the buffer solution in the receptacle (2);
- the closure of the receptacle (2) by screwing the cap (A) on said receptacle (2);
- the screwing of the capsule (4) on the threading of the safety ring nut (6);
- the breakage of the stopper (7) of the safety ring nut (6);
- the full tightening of the capsule (4) on the piercing cap (3);
- the breakage of the protective film (5) adhering to the base of the capsule (4);
- the spillage inside the container (2) of the formalin liquid, which mixes with the buffer solution;
- the transport of the container (1) to the analysis laboratory.

The materials and dimensions of the invention as above described, illustrated in the accompanying drawings and claimed below, may be any depending on the need. Moreover all details are replaceable with other technically equivalent ones, without departing from the scope of this patent application.

## Claims

1. Disposable container (1) for the preservation and transport of human tissue samples or organs to be subjected to histological examination comprising: a receptacle (2) containing a buffer solution, a piercing cap (3), a capsule (4) containing formalin sealed by means of a protective film (5), a safety ring nut (6) inserted between the base of the piercing cap (3) and the capsule (4)
**characterised in that**:
- said container is suitable for containing 30 or 90 ml;
- said capsule (4) contains formalin concentrated at 40% and 1/10 of the buffer solution;
- said piercing cap (3) is provided with threading;
- it is equipped with a stopper (7) positioned on the threading of the ring nut (6) to safeguard the protective film (5) adhering to the capsule (4).

2. Disposable container (1) of 30 or 90 ml according to claim 1 **characterised in that** the piercing of the protective film (5) occurs with the breakage of the stopper (7) due to the screwing of the capsule (4) on the threading of the ring nut (6).

3. Disposable container (1) of 30 or 90 ml according to claim 1, **characterised in that** it can horizontally contain a disposable plastic box.

4. Method for the preservation and transport of human tissue samples or organs comprising the following steps:
- opening of a disposable container (1) suitable for containing 30 or 90 ml by unscrewing a receptacle (2) from a cap (A) formed by the set of a safety ring nut (6), piercing cap (3) and a capsule (4) containing formalin concentrated at 40% and 1/10 of the buffer solution;
- introduction inside the receptacle (2), containing the buffer solution, of a needle of a device with the tissue sample taken from the patient;
- detachment of the tissue sample by the presence of the buffer solution in the receptacle (2);
- closure of the receptacle (2) by screwing the cap (A) on said receptacle (2);
- screwing of the capsule (4) on the threading of the safety ring nut (6);
- breakage of a stopper (7) positioned on the threading of the ring nut (6) to safeguard a protective film (5) adhering to the capsule (4);
- full tightening of the capsule (4) on the piercing cap (3);
- breakage of the protective film (5) adhering to the base of the capsule (4);
- spillage inside the container (2) of the formalin liquid, which mixes with the buffer solution;
- transport of the container (1) to the analysis laboratory.

## Patentansprüche

1. Einwegbehälter (1) zur Konservierung und Transport von Proben oder Organen menschlichen Gewebes, die einer histologischen Analyse unterzogen werden sollen, umfassend:
ein Gefäß (2), das eine Pufferlösung enthält, eine Durchstechkappe (3), eine Kapsel (4) die Formalin enthält und
mittels einem Schutzfilm (5) versiegelt ist, eine Mutter (6) eines Sicherheitsrings, der zwischen dem Boden der Durchstechkappe (3) und der Kapsel (4) eingesetzt ist.
**dadurch gekennzeichnet, dass**
- der Behälter ist für 30 oder 90 ml geeignet;
- die Kapsel (4) enthält 40%-konzentriertes Formalin und 1/10 der Pufferlösung;
- die genannte Durchstechkappe (3) ist mit einem Gewinde versehen;
- sie ist mit einem Anschlag (7) versehen, der auf dem Gewinde der Ringmutter (6) positioniert ist, um den Schutzfilm (5) zu schützen, der an der Kapsel (4) haftet.

2. Einwegbehälter (1) von 30 oder 90 ml nach Anspruch 1 **dadurch gekennzeichnet, dass** die Perforation des Schutzfilms (5) beim Aufbrechen des Anschlags (7) aufgrund des Aufschraubens der Kapsel (4) auf das Gewinde der Ringmutter (6) erfolgt.

3. Einwegbehälter (1) von 30 oder 90 ml nach Anspruch 1, **dadurch gekennzeichnet, dass** er horizontal eine Einweg-Kunststoffbox enthalten kann.

4. Verfahren zur Konservierung und Transport von Proben oder Organen menschlichen Gewebes, umfassend die folgenden Vorgänge:
- Öffnen eines Einwegbehälters (1), der für 30 oder 90 ml geeignet ist, durch Abschrauben eines Gefäßes (2) von einem Stopfen (A), der aus einer Mutter (6) eines Sicherheitsrings, einer Durchstechkappe (3) und einer Kapsel (4) besteht, die 40%-konzentriertes Formalin und 1/10 der Pufferlösung, enthält;
- Einführen ins Gefäß (2), das die Pufferlösung enthält, einer Nadel mit der dem Patienten entnommenen Gewebeprobe;
- Trennen der Gewebeprobe in Gegenwart der Pufferlösung im Gefäß (2);
- Verschließen des Gefäßes (2) durch Aufschrauben des Deckels (A) auf dem Gefäß (2);
- Aufschrauben der Kapsel (4) auf das Gewinde der Mutter (6) des Sicherungsrings;
- Brechen eines Anschlags (7), der auf dem Gewinde der Ringmutter (6) positioniert ist, um einen Schutzfilm (5) zu sichern, der an der Kapsel (4) haftet;
- vollständiges Verschließen der Kapsel (4) auf die Durchstechkappe (3);
- Brechen des Schutzfilms (5), der am Boden der Kapsel (4) haftet;
- Gießen von flüssigem Formalin ins Gefäß (2), das mit der Pufferlösung gemischt wird
- Transport des Behälters (1) zum Analyselabor.

## Revendications

1. Récipient jetable (1) pour le stockage et le transport d'échantillons ou d'organes de tissus humains à soumettre à une analyse histologique, comprenant: un récipient (2) contenant une solution tampon, un capuchon de perçage (3), une capsule (4) contenant du formol scellé au moyen d'un film protecteur (5), un écrou (6) d'un anneau de sécurité inséré entre la base du capuchon de perçage (3) et la capsule (4)
**caractérisé par le fait que**
- ledit récipient est adapté pour contenir 30 ou 90 ml;
- ladite capsule (4) contient du formol concentré à 40% et 1/10 de la solution tampon;
- ledit capuchon de perçage (3) est muni d'un filetage;
- il est muni d'une butée (7) positionnée sur le filetage de l'écrou (6) d'anneau, afin de protéger le film protecteur (5) qui adhère à la capsule (4).

2. Récipient jetable (1) de 30 ou 90 ml selon la revendication 1 **caractérisé en ce que** la perforation du film protecteur (5) se produit lors de la rupture de la butée (7) due au vissage de la capsule (4) sur le filetage de l'écrou (6) d'anneau.

3. Récipient jetable (1) de 30 ou 90 ml selon la revendication 1, **caractérisé en ce qu'**il peut contenir horizontalement une boîte en plastique jetable.

4. Méthode de conservation et de transport d'échantillons ou d'organes de tissus humains comprenant les opérations suivantes:
- ouverture d'un récipient jetable (1) apte à contenir 30 ou 90 ml en dévissant un récipient (2) d'un capuchon (A) formé d'une série constituée d'un écrou (6) d'une bague de sécurité, d'un capuchon de perçage (3) et d'une capsule (4) contenant du formol concentré à 40% et 1/10 de la solution tampon;
- introduction dans le récipient (2) contenant la solution tampon d'une aiguille d'un dispositif avec l'échantillon de tissu prélevé sur le patient;
- séparation de l'échantillon de tissu en présence de la solution tampon dans le récipient (2) ;
- fermeture du récipient (2) en vissant le bouchon (A) sur ledit récipient (2);
- vissage de la capsule (4) sur le filetage de l'écrou (6) de la bague de sécurité;
- rupture d'une butée (7) positionnée sur le filetage de l'écrou (6) de la bague pour protéger un film protecteur (5) qui adhère à la capsule (4) ;
- scellement complet de la capsule (4) du capuchon de perçage (3) ;
- rupture du film protecteur (5) qui adhère à la base de la capsule (4) ;
- versage dans le récipient (2) du formol liquide, qui est mélangé à la solution tampon ;
- transport du conteneur (1) vers le laboratoire d'analyse.
